# EUROPEAN PATENT APPLICATION

(11) **EP 2 517 708 A1**
(43) Date of publication of application: **31.10.2012**
(21) Application number: 10839542.7
(22) Date of filing: 24.12.2010
(51) Int. Cl.: A61K 31/404, A61P 35/00, C07D 403/06

(54) **METHOD FOR PREDICTING THERAPEUTIC EFFECT OF CHEMOTHERAPY ON HEPATOCELLULAR CARCINOMA PATIENT**

(30) Priority: 25.12.2009 JP 2009295913
(71) Applicant: Taiho Pharmaceutical Co., Ltd., Chiyoda-ku, Tokyo 101-8444 (JP)
(72) Inventor: OKA Toshinori, Tokushima-shi Tokushima 771-0194 (JP)
(74) Representative: Schrell, Andreas
(86) International application number: PCT/JP2010/073299
(87) International publication number: WO 2011/078312

(57) **Abstract**

The present invention provides a method for predicting therapeutic effects of chemotherapy on hepatocellular carcinoma patients who have been treated with transarterial embolization and an antitumor agent for treating cancer patients who have been predicted to have a high probability of obtaining sufficient therapeutic effects from such chemotherapy. The present invention also provides a method for predicting therapeutic effects of chemotherapy on hepatocellular carcinoma patients who have been treated with transarterial embolization using the expression level of PDGF-BB or IL-8 as an indicator and an antitumor agent for treating cancer patients who have been predicted to have a high probability of obtaining therapeutic effects by such prediction method.

SEQUENCE LISTING

<110>  TAIHO PHARMACEUTICAL CO., LTD.

<120>  A method of predicting therapeutic effects of chemotherapy in
       hepatocarcinoma having been treated with trancecatheter arterial
       chemoembolization

<130>  PH-4633-PCT

<150>  JP 2009-295913
<151>  2009-12-25

<160>  4

<170>  PatentIn version 3.4

<210>  1
<211>  1833
<212>  DNA
<213>  Homo sapiens

<400>  1
ctcgctgcaa agagaaaacc ggagcagccg cagctcgcag ctcgcagctc gcagcccgca      60

gcccgcagag gacgcccaga gcggcgagca ggcgggcaga cggaccgacg gactcgcgcc     120

gcgtccacct gtcggccggg cccagccgag cgcgcagcgg gcacgccgcg cgcgcggagc     180

agccgtgccc gccgcccggg ccccgcgcca gggcgcacac gctcccgccc ccctacccgg     240

cccgggcggg agtttgcacc tctccctgcc cgggtgctcg agctgccgtt gcaaagccaa     300

ctttggaaaa agtttttggg ggagacttgg gccttgaggt gcccagctcc gcgctttccg     360

gattttgggg gcctttccag aaaatgttgc aaaaaagcta agccggcggg cagaggaaaa     420

cgcctgtagc cggcgagtga agacgaacca tcgactgccg tgttcctttt cctcttggag     480

gttggagtcc cctgggcgcc cccacacggc tagacgcctc ggctggttcg cgacgcagcc     540

ccccggccgt ggatgctcac tcgggctcgg gatccgccca ggtagcggcc tcggacccag     600

gtcctgcgcc caggtcctcc cctgcccccc agcgacggag ccggggccgg gggcggcggc     660

gcccgggggc catgcgggtg agccgcggct gcagaggcct gagcgcctga tcgccgcgga     720

cccgagccga gcccaccccc ctccccagcc ccccaccctg gccgcggggg cggcgcgctc     780

gatctacgcg tccggggccc cgcggggccg ggcccggagt cggcatgaat cgctgctggg     840

cgctcttcct gtctctctgc tgctacctgc gtctggtcag cgccgagggg gaccccattc     900

ccgaggagct ttatgagatg ctgagtgacc actcgatccg ctcctttgat gatctccaac     960

gcctgctgca cggagacccc ggagaggaag atggggccga gttggacctg aacatgaccc    1020

gctcccactc tggaggcgag ctggagagct tggctcgtgg aagaaggagc ctgggttccc    1080

tgaccattgc tgagccggcc atgatcgccg agtgcaagac gcgcaccgag gtgttcgaga    1140

tctcccggcg cctcatagac cgcaccaacg ccaacttcct ggtgtggccg ccctgtgtgg    1200

aggtgcagcg ctgctccggc tgctgcaaca accgcaacgt gcagtgccgc cccacccagg     1260

tgcagctgcg acctgtccag gtgagaaaga tcgagattgt gcggaagaag ccaatcttta 1320

agaaggccac ggtgacgctg gaagaccacc tggcatgcaa gtgtgagaca gtggcagctg 1380

cacggcctgt gacccgaagc ccggggggtt cccaggagca gcgagccaaa acgccccaaa 1440

ctcgggtgac cattcggacg gtgcgagtcc gccggccccc caagggcaag caccggaaat 1500

tcaagcacac gcatgacaag acggcactga aggagaccct tggagcctag gggcatcggc 1560

aggagagtgt gtgggcaggg ttatttaata tggtatttgc tgtattgccc ccatggggtc 1620

cttggagtga taatattgtt tccctcgtcc gtctgtctcg atgcctgatt cggacggcca 1680

atggtgcttc ccccacccct ccacgtgtcc gtccaccctt ccatcagcgg gtctcctccc 1740

agcggcctcc ggcgtcttgc ccagcagctc aagaaaaaaa aaaaaaaaaa aaaaaaaaaa 1800

aaaaaaaaaa aaaaaaaaaa aaaaaaaaaa aaa 1833

<210> 2
<211> 241
<212> PRT
<213> Homo sapiens

<400> 2

<210>  3
<211>  1605
<212>  DNA
<213>  Homo sapiens

<400>  3
gaagaaacca ccggaaggaa ccatctcact gtgtgtaaac atgacttcca agctggccgt      60

ggctctcttg gcagccttcc tgatttctgc agctctgtgt gaaggtgcag ttttgccaag     120

gagtgctaaa gaacttagat gtcagtgcat aaagacatac tccaaacctt tccaccccaa     180

atttatcaaa gaactgagag tgattgagag tggaccacac tgcgccaaca cagaaattat     240

tgtaaagctt tctgatggaa gagagctctg tctggacccc aaggaaaact gggtgcagag     300

ggttgtggag aagtttttga agagggctga gaattcataa aaaaattcat tctctgtggt     360

atccaagaat cagtgaagat gccagtgaaa cttcaagcaa atctacttca acacttcatg     420

tattgtgtgg gtctgttgta gggttgccag atgcaataca agattcctgg ttaaatttga     480

atttcagtaa acaatgaata gtttttcatt gtaccatgaa atatccagaa catacttata     540

tgtaaagtat tatttatttg aatctacaaa aaacaacaaa taatttttaa atataaggat     600

tttcctagat attgcacggg agaatataca aatagcaaaa ttgaggccaa gggccaagag     660

aatatccgaa ctttaatttc aggaattgaa tgggtttgct agaatgtgat atttgaagca     720

tcacataaaa atgatgggac aataaatttt gccataaagt caaatttagc tggaaatcct     780

ggattttttt ctgttaaatc tggcaaccct agtctgctag ccaggatcca caagtccttg     840

ttccactgtg ccttggtttc tcctttattt ctaagtggaa aaagtattag ccaccatctt     900

acctcacagt gatgttgtga ggacatgtgg aagcacttta agttttttca tcataacata     960

aattattttc aagtgtaact tattaaccta tttattattt atgtatttat ttaagcatca 1020

aatatttgtg caagaatttg gaaaaataga agatgaatca ttgattgaat agttataaag 1080

atgttatagt aaatttattt tattttagat attaaatgat gttttattag ataaatttca 1140

atcagggttt ttagattaaa caaacaaaca attgggtacc cagttaaatt ttcatttcag 1200

ataaacaaca aataattttt tagtataagt acattattgt ttatctgaaa ttttaattga 1260

actaacaatc ctagtttgat actcccagtc ttgtcattgc cagctgtgtt ggtagtgctg 1320

tgttgaatta cggaataatg agttagaact attaaaacag ccaaaactcc acagtcaata 1380

ttagtaattt cttgctggtt gaaacttgtt tattatgtac aaatagattc ttataatatt 1440

atttaaatga ctgcattttt aaatacaagg ctttatattt ttaactttaa gatgttttta 1500

tgtgctctcc aaattttttt tactgtttct gattgtatgg aaatataaaa gtaaatatga 1560

aacatttaaa atataatttg ttgtcaaagt aaaaaaaaaa aaaaa 1605

<210> 4
<211> 99
<212> PRT
<213> Homo sapiens

<400> 4

Met Thr Ser Lys Leu Ala Val Ala Leu Leu Ala Ala Phe Leu Ile Ser
1               5                   10                  15

Ala Ala Leu Cys Glu Gly Ala Val Leu Pro Arg Ser Ala Lys Glu Leu
            20                  25                  30

Arg Cys Gln Cys Ile Lys Thr Tyr Ser Lys Pro Phe His Pro Lys Phe
        35                  40                  45

Ile Lys Glu Leu Arg Val Ile Glu Ser Gly Pro His Cys Ala Asn Thr
    50                  55                  60

Glu Ile Ile Val Lys Leu Ser Asp Gly Arg Glu Leu Cys Leu Asp Pro
65                  70                  75                  80

Lys Glu Asn Trp Val Gln Arg Val Val Glu Lys Phe Leu Lys Arg Ala
                85                  90                  95

Glu Asn Ser

## Description

### Technical Field

The present invention relates to a method for predicting therapeutic effects of chemotherapy with an antitumor agent comprising (Z)-5-[(1,2-dihydro-2-oxo-3H-indol-3-ylidene)methyl]2,4-dimethyl-1H-pyrro 1-3-propanoic acid (hereinafter referred to as "TSU-68") or a salt thereof on hepatocellular carcinoma patients who have been treated with transarterial embolization and an antitumor agent for treating cancer patients who have been predicted to have a high probability of obtaining sufficient therapeutic effects from such chemotherapy.

### Background Art

There are a variety of hepatocellular carcinoma treatment methods such as surgical resection, transarterial embolization (hereinafter referred to as "TAE"), percutaneous ablation therapy (PAT) (e.g., percutaneous ethanol injection therapy (PEIT) or radiofrequency ablation (RFA)), chemotherapy, radiation therapy, and liver transplantation. TAE is a surgical treatment method comprising injecting an embolic material such as gelatin sponge into the hepatic artery of a hepatocellular carcinoma patient to embolize the nutrient artery so as to cause necrosis of hepatocellular carcinoma in a selective manner. TAE also includes transcatheter arterial chemo-embolization (hereinafter referred to as "TACE") for administering an antitumor agent to lesions during surgical treatment. TAE plays an important role in hepatocellular carcinoma treatment because TAE is the most frequently used treatment method for initial treatment cases and recurring cases, and TAE is particularly recommended for cases in which surgical resection or PAT is not feasible as well as recurring cases (Non-Patent Literature 1). Meanwhile, TAE is problematic in that TAE rarely results in complete tumor necrosis after treatment, and thus, in many cases, tumor remains in the peripheral zone of the treatment site, causing recurrence or metastasis several months after TAE. Thus, if inhibition of such recurrence or metastasis becomes possible, it can contribute to further extension of life expectancy.

Hitherto, there have been attempts to develop a variety of antitumor agents for hepatocellular carcinoma. For example, there are anthracycline antitumor agents, platinum antitumor agents, alkaloid antitumor agents, nucleoside antimetabolites, and the like. However, it is said that administration of such antitumor agents is not recommended according to treatment guidelines (Non-Patent Literature 1 and Non-Patent Literature 2).

In recent years, the elucidation of cancer at the molecular biological level has led to discovery of a variety of molecular-targeted antitumor agents that target specific molecules such as angiogenesis-related factors and cell-growth-related factors expressed in cancer cells. Such molecular-targeted antitumor agents differ from conventional antitumor agents in terms of action mechanisms and toxicity profiles. Thus, they have been gaining attention because of their potential to contribute to treatment of hepatocellular carcinoma.

TSU-68, which is known as a molecular-targeted antitumor agent, is a low-molecular-weight compound. It inhibits tyrosine phosphorylation of Flk-1 (also referred to as "KDR"), which is a vascular endothelial growth factor (hereinafter referred to as "VEGF") receptor, so as to inhibit angiogenesis in tumor tissue for interruption of oxygen and nutrition supply, thereby inhibiting tumor growth and metastasis. In addition, it has been confirmed *in vitro* that the low-molecular-weight compound also inhibits tyrosine phosphorylation of platelet-derived growth factor (hereinafter referred to as "PDGF") receptors, FGF receptors, and the like, which are involved in intracellular signal transduction, as well as the VEGF receptors. As a result of examination of antitumor effects of the oral administration of TSU-68 alone on *in vivo* nude mouse models into which various human cancer cell lines had been subcutaneously implanted, TSU-68 was confirmed to be effective for inhibiting tumor growth in lung cancer, colon cancer, ovarian cancer, and the like (Non-Patent Literature 3). It has been reported that TSU-68 has treatment effects on some types of cancer such as hepatocellular carcinoma in clinical studies (Non-Patent Literature 4).

In addition to TSU-68, there are known VEGF receptor inhibitors such as sunitinib and sorafenib. There have been attempts to conduct clinical studies to use sunitinib and sorafenib for adjuvant therapy after TAE; however, it still has not been reported whether treatment effects have been confirmed. Antitumor agents that have therapeutic effects against recurrence after TAE with certainty have been awaited (Non-Patent Literature 5).

As described above, no appropriate therapeutic method has been established as the standard therapy for the hepatocellular carcinoma treatment system. In particular, no therapeutic method effective after TAE has yet been established.

### Citation List

### Non-Patent Literature

Non-Patent Literature 1: National Comprehensive Cancer Network Clinical Practice Guidelines in Oncology Hepatobiliary Cancers v.2, 2009
Non-Patent Literature 2: Ann Surg Oncol 15 (4): 1008-14, 2008
Non-Patent Literature 3: Cancer Res 60 (15): 4152-60, 2000
Non-Patent Literature 4: European Journal of Cancer Supplements, 6 (12): 17 #43, 2008
Non-Patent Literature 5: BMC Cancer 8:349,2008

### Summary of Invention

### Technical Problem

An object of the present invention is to provide a method for predicting therapeutic effects of chemotherapy with an antitumor agent comprising TSU-68 or a salt thereof on a hepatocellular carcinoma patient who has been treated with TAE and an antitumor agent for treating a cancer patient who has been predicted to have a high probability of obtaining sufficient therapeutic effects from such chemotherapy.

### Solution to Problem

The present inventors conducted intensive studies on chemotherapy for hepatocellular carcinoma patients who had been treated with TAE. As a result, the present inventors have devised a method for predicting therapeutic effects of chemotherapy with TSU-68 using, as an indicator, the expression level of a BB isoform of PDGF (hereinafter referred to as "PDGF-BB") or interleukin-8 (hereinafter referred to as "IL-8"). This has led to the completion of the present invention. Hitherto, it has been completely unknown for the expression level of PDGF-BB or IL-8 to be used as an indicator for selecting such therapeutic method for a hepatocellular carcinoma patient who has been treated with TAE.

Specifically, the present invention is described as below.

[1] A method for predicting therapeutic effects of chemotherapy with an antitumor agent comprising (Z)-5-[(1,2-dihydro-2-oxo-3H-indol-3-ylidene)methyl]2,4-dimethyl-1H-pyrro 1-3-propanoic acid or a salt thereof on a hepatocellular carcinoma patient who has been treated with transarterial embolization, which comprises the following steps (1) to (3):
(1) a step of determining the expression level of PDGF-BB or IL-8 contained in a biological sample collected from the patient;
(2) a step of comparing the expression level of PDGF-BB or IL-8 determined in step (1) with a predetermined corresponding cut-off point; and
(3) a step of predicting that the patient has a high probability of obtaining sufficient therapeutic effects from chemotherapy with an antitumor agent comprising (Z)-5-[(1,2-dihydro-2-oxo-3H-indol-3-ylidene)methyl]2,4-dimethyl-1H-pyrro 1-3-propanoic acid or a salt thereof, if the expression level of PDGF-BB or IL-8 is higher than the cut-off point as a result of comparison in step (2).

[2] The method of [1], wherein transarterial embolization is transarterial chemoembolization.

[3] An antitumor agent comprising (Z)-5-[(1,2-dihydro-2-oxo-3H-indol-3-ylidene)methyl]2,4-dimethyl-1H-pyrro 1-3-propanoic acid or a salt thereof, which is administered to a cancer patient who has been predicted to have a high probability of obtaining sufficient therapeutic effects from chemotherapy with an antitumor agent comprising (Z)-5-[(1,2-dihydro-2-oxo-3H-indol-3-ylidene)methyl]2,4-dimethyl-1H-pyrro 1-3-propanoic acid or a salt thereof according to the method of [1] or [2].

[4] An antitumor agent kit including the antitumor agent of [3] and instructions for use that specify administration of (Z)-5-[(1,2-dihydro-2-oxo-3H-indol-3-ylidene)methyl]2,4-dimethyl-1H-pyrro 1-3-propanoic acid or a salt thereof to a cancer patient who has been predicted to have a high probability of obtaining sufficient therapeutic effects from chemotherapy with an antitumor agent comprising (Z)-5-[(1,2-dihydro-2-oxo-3 H-indol-3-ylidene)methyl]2,4-dimethyl-1H-pyrro 1-3-propanoic acid or a salt thereof according to the method of [1] or [2].

[5] A method for treating hepatocellular carcinoma of a hepatocellular carcinoma patient who has been treated with transarterial embolization, which comprises administering an antitumor agent comprising (Z)-5-[(1,2-dihydro-2-oxo-3H-indol-3-ylidene)methyl]2,4-dimethyl-1H-pyrro 1-3-propanoic acid or a salt thereof to a cancer patient who has been predicted to have a high probability of obtaining sufficient therapeutic effects from chemotherapy with an antitumor agent comprising (Z)-5-[(1,2-dihydro-2-oxo-3H-indol-3-ylidene)methyl]2,4-dimethyl-1H-pyrro 1-3-propanoic acid or a salt thereof according to the method of [1] or [2].

[6]
(Z)-5-[(1,2-dihydro-2-oxo-3H-indol-3-ylidene)methyl]2,4-dimethyl-1H -pyrrol-3-propanoic acid or a salt thereof, which is used for a cancer patient who has been predicted to have a high probability of obtaining sufficient therapeutic effects from chemotherapy with an antitumor agent comprising (Z)-5-[(1,2-dihydro-2-oxo-3H-indol-3-ylidene)methyl]2,4-dimethyl-1H-pyrro 1-3-propanoic acid or a salt thereof according to the method of [1] or [2] in order to treat hepatocellular carcinoma of a hepatocellular carcinoma patient who has been treated with transarterial embolization.

This description includes part or all of the contents as disclosed in the description and/or drawings of Japanese Patent Application No. 2009-295913, which is a priority document of the present application.

### Advantageous Effects of Invention

The prediction method of the present invention enables selection of effective chemotherapy which can realize remarkably excellent therapeutic effects (and especially life-extending effects (e.g., extension of progression-free survival and suppression of recurrence/metastasis)); that is to say, chemotherapy with an antitumor agent comprising TSU-68 or a salt thereof for hepatocellular carcinoma patients who have been treated with TAE. Specifically, chemotherapy by which further excellent therapeutic effects are obtained can be adequately provided only to hepatocellular carcinoma patients who can be expected to obtain therapeutic effects therefrom. Hence, unnecessary chemotherapy can be omitted and thus burdens on patients can be reduced. Further, the present invention is preferable in terms of medical cost efficiency.

### Description of Embodiments

The prediction method of the present invention is intended to predict therapeutic effects of chemotherapy with an antitumor agent comprising TSU-68 or a salt thereof on a hepatocellular carcinoma patient who has been treated with TAE based on the expression level of PDGF-BB or IL-8 of the patient. More specifically, when the PDGF-BB or IL-8 expression level of the patient is compared with the predetermined corresponding cut-off point and found to be higher than the cut-off point, it is predicted that the patient will have a high probability of obtaining sufficient therapeutic effects from chemotherapy with an antitumor agent comprising TSU-68 or a salt thereof.

The phrase "obtaining sufficient therapeutic effects from chemotherapy" indicates that a hepatocellular carcinoma patient who has been treated with TAE obtains therapeutic effects (and especially life-extending effects) that are significantly more excellent than those obtained without the agent. In the present invention, "therapeutic effects" are used to assess tumor reduction effects, life-extending effects, and the like in a comprehensive manner. In particular, the presence of life-extending effects is an important indicator. Life-extending effects can be evaluated in a comprehensive manner based on extension of progression-free survival, suppression of recurrence or metastasis, or the like.

"TSU-68" is a known inhibitor of a VEGF receptor or the like denoted as (Z)-5-[(1,2-dihydro-2-oxo-3H-indol-3-ylidene)methyl]2,4-dimethyl-1H-pyrro 1-3-propanoic acid. TSU-68 is known to have tumor-growth-inhibiting effects on solid cancers such as liver cancer, lung cancer, colon cancer, and ovarian cancer. TSU-68 or a salt thereof can be produced by a known method, such as the method of JP Patent Publication (Kohyo) No. 2002-516310 A.

A salt of TSU-68 is not particularly limited as long as it is a pharmaceutically acceptable salt. Examples thereof include salts obtained by reacting TSU-68 with inorganic acids such as hydrochloric acid, hydrobromic acid, sulfuric acid, nitric acid, and phosphoric acid and organic acids such as methanesulfonic acid, ethanesulfonic acid, p-toluenesulfonic acid, and salicylic acid.

The administration form of an antitumor agent comprising TSU-68 or a salt thereof is not particularly limited, and thus it can be adequately selected depending on treatment purposes. Specific examples thereof include oral agents (e.g., tablets, coated tablets, powders, granules, capsules, and liquids), parenteral injections, suppositories, patches, and ointments. Oral agents are preferable.

The antitumor agent comprising TSU-68 or a salt thereof can be prepared by a conventionally known method using a pharmaceutically acceptable carrier. Examples of such carrier include a variety of carriers widely used for general drugs, such as excipients, binders, disintegrators, lubricants, diluents, solubilizing agents, suspending agents, isotonizing agents, pH adjusters, buffers, stabilizers, colorants, corrigents, and flavoring agents.

Examples of excipients include lactose, sucrose, sodium chloride, glucose, maltose, mannitol, erythritol, xylitol, maltitol, inositol, dextran, sorbitol, albumin, urea, starch, calcium carbonate, kaoline, crystalline cellulose, silicic acid, methylcellulose, glycerine, sodium alginate, gum arabic, and mixtures thereof. Examples of lubricants include purified talc, stearate, borax, polyethylene glycol, and mixtures thereof. Examples of binders include simple syrups, glucose solutions, starch solutions, gelatin solutions, polyvinyl alcohol, polyvinyl ether, polyvinyl pyrrolidone, carboxymethylcellulose, shellac, methylcellulose, ethylcellulose, water, ethanol, potassium phosphate, and mixtures thereof. Examples of disintegrators include dry starch, sodium alginate, agar powder, laminaran powder, sodium hydrogen carbonate, calcium carbonate, polyoxyethylene sorbitan fatty acid esters, sodium lauryl sulfate, stearic acid monoglyceride, starch, lactose, and mixtures thereof. Examples of diluents include water, ethyl alcohol, macrogol, propylene glycol, ethoxylatedisostearyl alcohol, polyoxygenated isostearyl alcohol, polyoxyethylene sorbitan fatty acid esters, and mixtures thereof. Examples of stabilizers include sodium pyrosulfite, ethylenediamine tetraacetate, thioglycollic acid, thiolactic acid, and mixtures thereof. Examples of isotonizing agents include sodium chloride, boric acid, glucose, glycerine, and mixtures thereof. Examples of pH adjusters and buffers include sodium citrate, citric acid, sodium acetate, sodium phosphate, and mixtures thereof. Examples of soothing agents include procaine hydrochloride, lidocaine hydrochloride, and mixtures thereof.

The administration dose of the antitumor agent used in chemotherapy for a hepatocellular carcinoma patient who has been treated with TAE can be adequately determined depending on conditions such as patient age, weight, and sex, disease stage, the presence or absence of metastasis, the history of treatment, and the use or non-use of a different antitumor agent. For example, it is preferably 100 to 3000 mg/day, more preferably 200 to 1600 mg/day, and particularly preferably 400 to 800 mg/day. In addition, the administration schedule of the antitumor agent can be adequately determined depending on conditions such as patient age, weight, and sex, disease stage, the presence or absence of metastasis, and the history of treatment. For example, it is preferable to administer the antitumor agent once daily or two to four times daily in separate doses on consecutive days.

Patients for whom the present invention is used are hepatocellular carcinoma patients who have been treated with TAE. TAE is a surgical treatment method comprising injecting an embolic material such as gelatin sponge into the hepatic artery that supplies nutrition to tumor cells to embolize the nutrient artery so as to cause necrosis of hepatocellular carcinoma in a selective manner (National Comprehensive Cancer Network Clinical Practice Huidelines in Oncology Hepatobiliary Cancers v.2, 2009). In addition, TAE of the present invention also includes TACE for administration of antitumor agents. Examples of TACE include a method wherein a mixture of an oily contrast agent such as lipiodol and antitumor agent(s) is injected into the nutrient artery prior to injection of an embolic material and a method wherein drug (antitumor agent)-eluting beads are used instead of antitumor agents (i.e., DEB-TACE) (J Hepatol. 2007 Mar; 46(3): 474-81). Further, antitumor agents used for TACE are not particularly limited as long as they can be used for hepatocellular carcinoma. Examples thereof include doxorubicin, epirubicin, and cisplatin.

PDGF is a known growth factor mainly involved in the control of migration and growth of mesenchymal cells (such as fibroblasts, smooth muscle cells, and glial cells). Four PDGF types for humans are known, namely, PDGFA, PDGFB, PDGFC, and PDGFD. PDGFA and PDGFB each have a homodimer or heterodimer structure formed with a disulfide bond, resulting in three different isoforms (PDGF-AA, PDGF-AB, and PDGF-BB). The indicator used in the present invention is PDGF-BB, which is a homodimer of PDGFB. The nucleotide sequence and the amino acid sequence of human PDGFB are known in the art. For example, the nucleotide sequence and the amino acid sequence have been deposited with accession nos. BC077725 and AAH77725 (SEQ ID NOS: 1 and 2), respectively, in GenBank.

IL-8 is a cytokine that functions for intercellular signal transduction and is known as a neutrophil chemoattractant. The nucleotide sequence and the amino acid sequence of human IL-8 are known in the art. For example, the nucleotide sequence and the amino acid sequence have been deposited with accession nos. BC013615 and AAH13615 (SEQ ID NOS: 3 and 4), respectively, in GenBank.

In addition, according to the present invention, the nucleotide sequences of human PDGFB and human IL-8 include a nucleotide sequence that has a deletion, substitution, addition, or insertion of one or more nucleotides with respect to any of the above known nucleotide sequences and encodes a protein having the activity of the corresponding protein. The phrase "one or more" used herein refers to, but is not particularly limited to, for example, 1 to 20 nucleotides, preferably 1 to 10 nucleotides, more preferably 1 to 7 nucleotides, further preferably 1 to 5 nucleotides, and particularly preferably 1 to 3 nucleotides (or 1 nucleotide or 2 nucleotides). In addition, the nucleotide sequences of human PDGFB and human IL-8 include a nucleotide sequence that hybridizes under stringent conditions to a nucleotide sequence consisting of a sequence complementary to any of the above known nucleotide sequences and encodes a protein having the activity of the corresponding protein. Here, the term "stringent conditions" refers to conditions under which namely a specific hybrid is formed, but a non-specific hybrid is never formed. For example, such conditions comprise a sodium concentration of 10 mM to 300 mM and preferably 20 to 100 mM, and a temperature of 25°C to 70°C and preferably 42°C to 55°C. Further, the nucleotide sequences of human PDGFB and human IL-8 include a nucleotide sequence that encodes a protein comprising a nucleotide sequence having 80% or more, preferably 90% or more, and most preferably 95% or more homology to any of the above known nucleotide sequences when the homology is calculated using, for example, BLAST (the Basic Local Alignment Search Tool at the National Center for Biological Information) (based on, for example, default (i.e., initial setting) parameters) and having the activity of the corresponding protein.

In addition, according to the present invention, the amino acid sequences of human PDGFB and human IL-8 include an amino acid sequence that has a deletion, substitution, addition, or insertion of one or more amino acids with respect to any of the above known amino acid sequences and has the activity of the corresponding protein. Further, the amino acid sequences of human PDGFB and human IL-8 include an amino acid sequence having 80% or more, preferably 85% or more, more preferably 90% or more, for example, 93% or more, 95% or more, 97% or more, 98% or more, or 99% or more identity to any of the above known amino acid sequences when the identity is calculated using, for example, BLAST (based on, for example, default (i.e., initial setting) parameters) and having the activity of the corresponding protein.

According to the prediction method of the present invention, the expression levels of PDGF-BB and IL-8 can be determined using biological samples from patients. Examples of biological samples include body fluid (e.g., blood or urine), tissue, an extract of any thereof, and a culture product of collected tissue. In view of ease of sampling and the like, body fluid (and, in particular, blood such as pheripheral blood) is preferable. In addition, a method for collecting a biological sample can be adequately selected depending on the biological sample type. DNAs, RNAs, and proteins can be prepared from biological samples by conventionally known methods.

According to the prediction method of the present invention, the expression level of PDGF-BB or IL-8 is used as an indicator. The expression level may be the mRNA or protein expression level.

The mRNA expression level of PDGF-BB or IL-8 can be determined by a known gene expression level determination method such as Northern blotting, quantitative or semi-quantitative PCR (e.g., RT-PCR or real-time PCR), or an *in situ* hybridization method using probes or primers that specifically hybridize to mRNA of PDGFB or IL-8. The expression level can be evaluated based on the ratio of the expression level to the expression level of a protein/gene (e.g., a house keeping gene such as β-actin or an expression protein thereof) which is constantly expressed at a level within the certain range.

Meanwhile, the protein expression level of PDGF-BB or IL-8 can be determined by a known immunoassay method, such as enzyme immunoassay, radioimmunoassay, fluoroimmunoassay, ELISA, Western blotting, or immunohistochemical staining, using an antibody that specifically recognizes PDGF-BB or IL-8.

The above antibody is not particularly limited as long as it specifically recognizes PDGF-BB or IL-8 and thus it may be a monoclonal or polyclonal antibody or an antibody fragment such as a Fab fragment or a F(ab')2 fragment. Such antibody can be produced by a conventionally known method (see, for example, Current protocols in Molecular Biology edit. Ausubel et al. (1987), Publish. John Wiley and Sons. Section 11.12-11.13). In addition, the antibody may be a commercially available antibody. Alternatively, a commercially available kit for determining the expression level of PDGF-BB or IL-8 such as "Human PDGF-BB Immunoassay" (R&D systems) or a "Human IL8 EASIA kit" (BIOSOURCE EUROPE S.A.) can be used.

According to the prediction method of the present invention, when the expression level of PDGF-BB or IL-8 of a hepatocellular carcinoma patient who has been treated with TAE is higher than the predetermined corresponding cut-off point, the patient is predicted to have a high probability of obtaining remarkably excellent therapeutic effects from chemotherapy with an antitumor agent comprising TSU-68 or a salt thereof.

Cut-off points can be determined by a variety of statistical analysis methods based on the predetermined PDGF-BB or IL-8 expression level.

The cut-off point of PDGF-BB can be determined to be any of the values specified below. Here, the progression-free survival rate and the hazard ratio can be determined by conventional techniques.

1. Mean or median of PDGF-BB expression levels of hepatocellular carcinoma patients who have been treated with TAE or TACE.
2. Mean or median of PDGF-BB expression levels of hepatocellular carcinoma patients who have been treated with TAE or TACE in the TSU-68 administration group.
3. The PDGF-BB expression level for dividing hepatocellular carcinoma patients who have been treated with TAE or TACE into the PDGF-BB hyperexpression group and the PDGF-BB underexpression group, at which the difference between the 6-month-progression-free survival rate for the TSU-68 administration group of the PDGF-BB hyperexpression group and the 6-month-progression-free survival rate for the TSU-68 non-administration group of the PDGF-BB hyperexpression group reaches the maximum level or not less than a certain level (provided that the phrase "not less than a certain level" means that the difference in terms of the 6-month-progression-free survival rate is 20% or more, 25% or more, 30% or more, 40% or more, or 50% or more (e.g., 20% or more)).
4. The PDGF-BB expression level for dividing hepatocellular carcinoma patients who have been treated with TAE or TACE into the PDGF-BB hyperexpression group and the PDGF-BB underexpression group, at which the hazard ratio of the TSU-68 administration group of the PDGF-BB hyperexpression group to the TSU-68 non-administration group of the PDGF-BB hyperexpression group reaches the minimum level or not more than a certain level (provided that the phrase "not more than a certain level" means that the hazard ratio is 0.65 or less, 0.6 or less, 0.55 or less, or 0.5 or less (e.g., 0.6 or less)).
5. The PDGF-BB expression level for dividing hepatocellular carcinoma patients who have been treated with TAE or TACE into the PDGF-BB hyperexpression group and the PDGF-BB underexpression group, at which the difference between the 6-month-progression-free survival rate for the TSU-68 administration group of the PDGF-BB hyperexpression group and the 6-month-progression-free survival rate for the TSU-68 administration group of the PDGF-BB underexpression group reaches the maximum level or not less than a certain level (provided that the phrase "not less than a certain level" means that the difference in terms of the 6-month-progression-free survival rate is 20% or more, 25% or more, 30% or more, 40% or more, or 50% or more (e.g., 20% or more)).
6. The PDGF-BB expression level for dividing hepatocellular carcinoma patients who have been treated with TAE or TACE into the PDGF-BB hyperexpression group and the PDGF-BB underexpression group, at which the hazard ratio of the TSU-68 administration group of the PDGF-BB hyperexpression group to the TSU-68 administration group of the PDGF-BB underexpression group reaches the minimum level or not more than a certain level (provided that the phrase "not more than a certain level" means that the hazard ratio is 0.65 or less, 0.6 or less, 0.55 or less, or 0.5 or less, for example, 0.6 or less).
7. The expression level of PDGF-BB for dividing hepatocellular carcinoma patients who have been treated with TAE or TACE into the PDGF-BB hyperexpression group and the PDGF-BB underexpression group, at which the value obtained by subtracting the difference between the 6-month-progression-free survival rate for the TSU-68 administration group of the PDGF-BB underexpression group and the 6-month-progression-free survival rate for the TSU-68 non-administration group of the PDGF-BB underexpression group from the difference between the 6-month-progression-free survival rate for the TSU-68 administration group of the PDGF-BB hyperexpression group and the 6-month-progression-free survival rate for the TSU-68 non-administration group of the PDGF-BB hyperexpression group reaches the maximum level or not less than a certain level (provided that the phrase "not less than a certain level" means that the result obtained by subtraction is 10% or more, 20% or more, 30% or more, 40% or more, or 50% or more (e.g., 10% or more)).
8. The expression level of PDGF-BB for dividing hepatocellular carcinoma patients who have been treated with TAE or TACE into the PDGF-BB hyperexpression group and the PDGF-BB underexpression group, at which the value obtained by dividing the hazard ratio of the TSU-68 administration group of the PDGF-BB hyperexpression group to the TSU-68 non-administration group of the PDGF-BB hyperexpression group by the hazard ratio of the TSU-68 administration group of the PDGF-BB underexpression group to the TSU-68 non-administration group of the PDGF-BB underexpression group reaches the minimum level or not more than a certain level (provided that the phrase "not more than a certain level" means that the result obtained by division is 0.8 or less, 0.7 or less, 0.6 or less, 0.5 or less, or 0.4 or less (e.g., 0.8 or less)).

Similarly, the cut-off points for IL-8 can be obtained in the same manner as that above in the case of the cut-off points for PDGF-BB.

More specifically, as described in detail in the examples described below, when the protein expression level is determined using, for example, peripheral blood as a biological sample by the above cut-off point calculation method, the cut-off point of PDGF-BB is preferably 1480 to 2030 pg/ml and particularly preferably 1740 to 1960 pg/ml, and the cut-off point of IL-8 is preferably 2.1 to 10.5 pg/ml and particularly preferably 2.1 to 6.6 pg/ml.

Note that every cut-off point would vary depending on conditions such as types of assay items or assay methods, and thus it must be predetermined depending on such conditions. Cut-off points may vary depending on assay items (number of patients, patient age, sex, and weight, health state, disease state, and type of biological sample), assay methods (for which whether genes or proteins are assayed as expression products), assay conditions (e.g., sequences of primers or probes for determination of a gene expression product (mRNA), type of label, and antibody type and sensitivity if the expression product is a protein), and statistical techniques.

In another embodiment, the present invention relates to an antitumor agent comprising TSU-68 or a salt thereof for treating a cancer patient who has been predicted to have a high probability of obtaining sufficient therapeutic effects from chemotherapy with an antitumor agent comprising TSU-68 or a salt thereof. The antitumor agent is used in the aforementioned administration form.

In another embodiment, the present invention relates to an antitumor agent kit including TSU-68 or a salt thereof and instructions for use that specify administration of TSU-68 or a salt thereof to a cancer patient who has been predicted to have a high probability of obtaining sufficient therapeutic effects from chemotherapy with an antitumor agent comprising TSU-68 or a salt thereof according to the above prediction method. Here, "instructions for use" are not legally binding as long as they contain descriptions of TSU-68 administration methods/administration criteria. Specific examples thereof include package inserts and pamphlets. In addition, "instructions for use" may be printed on or attached to a package for an antitumor agent kit or may be enclosed together with an antitumor agent in the package for an antitumor agent kit.

In another embodiment, the present invention relates to a method for selecting a patient who has been predicted to have a high probability of obtaining sufficient therapeutic effects of chemotherapy with an antitumor agent comprising TSU-68 or a salt thereof from among hepatocellular carcinoma patients who have been treated with TAE. The method comprises the following steps (1) to (3):
(1) a step of determining the expression level of PDGF-BB or IL-8 contained in a biological sample collected from the patient;
(2) a step of comparing the expression level of PDGF-BB or IL-8 obtained in step (1) with the predetermined corresponding cut-off point; and
(3) a step of predicting that a patient whose PDGF-BB or IL-8 expression level has been found to be higher than the cut-off point as a result of comparison in step (2) is a patient having a high probability of obtaining sufficient therapeutic effects from chemotherapy with an antitumor agent comprising TSU-68 or a salt thereof, and approving treatment for such patient.

In another embodiment, the present invention relates to a method for treating a hepatocellular carcinoma patient who has been treated with TAE via chemotherapy with an antitumor agent comprising TSU-68 or a salt thereof. The method comprises the following steps (1) to (4):
(1) a step of determining the expression level of PDGF-BB or IL-8 contained in a biological sample collected from the patient;
(2) a step of comparing the expression level of PDGF-BB or IL-8 obtained in step (1) with the predetermined corresponding cut-off point;
(3) a step of predicting that a patient whose PDGF-BB or IL-8 expression level has been found to be higher than the cut-off point as a result of comparison in step (2) is a patient having a high probability of obtaining sufficient therapeutic effects from chemotherapy with an antitumor agent comprising TSU-68 or a salt thereof, and approving the patient as suitable to receive such chemotherapy; and
(4) a step of administering an antitumor agent comprising TSU-68 or a salt thereof to the patient approved in step (3).

The above cut-off points can be used as the cut-off points for PDGF-BB and IL-8 used in the above method for selection and treatment. In addition, steps (1) to (3) used in the method for selection and treatment can be carried out in the same manner as that used in the case of the aforementioned prediction method.

### Examples

The present invention is hereafter described in greater detail with reference to the following examples. Needless to say, the present invention is not limited to the examples.

### (Example 1) Calculation of cut-off points

A comparative clinical study was conducted for the TSU-68 administration group and the TSU-68 non-administration group consisting of cases diagnosed as hepatocellular carcinoma that were impossible to treat with hepatectomy and PAT and had been treated with TACE. The antitumor agent used herein for TACE was epirubicin.

The subject cases were randomly assigned to the TSU-68 administration group and the TSU-68 non-administration group. TSU-68 administration was initiated for cases assigned to the TSU-68 administration group within 2 weeks after TACE. TSU-68 was administered orally at a dose of 200 mg per administration twice daily after breakfast and dinner on consecutive days. No further cancer treatment was given to the TSU-68 non-administration group after TACE and thus only follow-up observation was carried out.

Progression-free survival was defined as survival within the period from the day of TACE designated as the initial date of reckoning to any of dates on which the following events was observed, whichever came first.

1. Date on which the presence of progressive disease (hereinafter referred to as "PD") was confirmed as a result of assessment of best overall response (date of testing).

2. Date on which a test subject died for any reason (date of death).

3. Date on which a test subject was assessed to have experienced clinically obvious deterioration of the disease or to be in need of post-TACE treatment even without confirmation of PD upon assessment of best overall response (date of assessment).

Best overall response was evaluated by measuring tumor diameters by CT and assaying tumor markers (AFP, PIVKA-II, and AFP-L3 fractions) according to New Guideline to Evaluate the Response to Treatment in Solid Tumor (hereinafter referred to as "RECIST") (J Natl Cancer Inst 92: 205, 2000).

Table 1 shows treatment outcomes for the TSU-68 administration group and the TSU-68 non-administration group consisting of hepatocellular carcinoma patients treated with TACE in this study.

**[Table 1]**

| Comparison of the TSU-68 administration group and the TSU-68 non-administration group in terms of therapeutic effects | | | | |
|---|---|---|---|---|
| Subject | Number of cases | 6-month-progression-free survival rate (%) | Difference between 6-month-progression-free survival rates (%) | Hazard ratio |
| TSU-68 administration group | 50 | 47 | 18 | 0.69 |
| TSU-68 non-administrati on group | 51 | 29 | | |

The expression levels of PDGF-BB and IL-8 in blood obtained before TSU-68 administration after TACE were determined using ELISA Kits as described below.

**[Table 2]**

| Manufacturer | Kit name |
|---|---|
| R&D systems | Human PDGF-BB Immunoassay |
| BIOSOURCE EUROPE S.A. | Human IL8 EASIA kit |

Cut-off points were determined by the statistical analysis technique described below based on the quantified PDGF-BB and IL-8 levels.

(1) The cut-off point for dividing patients into the PDGF-BB hyperexpression group and the PDGF-BB underexpression group was calculated so that the difference between the 6-month-progression-free survival rate for the TSU-68 administration group and the 6-month-progression-free survival rate for the TSU-68 non-administration group was 20% or more for patients in the PDGF-BB hyperexpression group. The cut-off point was 1480 to 2030 pg/ml.

(2) The cut-off point for dividing patients into the PDGF-BB hyperexpression group and the PDGF-BB underexpression group was calculated so that the hazard ratio of the TSU-68 administration group to the TSU-68 non-administration group was 0.6 or less for patients in the PDGF-BB hyperexpression group. The cut-off point was 1740 to 1960 pg/ml.

(3) The cut-off point for dividing patients into the IL-8 hyperexpression group and the IL-8 underexpression group was calculated so that the difference between the 6-month-progression-free survival rate for the TSU-68 administration group and the 6-month-progression-free survival rate for the TSU-68 non-administration group was 20% or more for cases in the IL-8 hyperexpression group. The cut-off point was 2.1 to 6.6 pg/ml.

(4) The cut-off point for dividing patients into the IL-8 hyperexpression group and the IL-8 underexpression group was calculated so that the hazard ratio of the TSU-68 administration group to the TSU-68 non-administration group was 0.6 or less for cases in the IL-8 hyperexpression group. The cut-off point was 2.1 to 10.5 pg/ml.

### (Example 2) Therapeutic effects on patients classified based on PDGF-BB or IL-8

The PDGF-BB hyperexpression group, the PDGF-BB underexpression group, the IL-8 hyperexpression group, and the IL-8 underexpression group were subjected to survival time analysis of the TSU-68 administration group and the TSU-68 non-administration group with the use of the cut-off points calculated in Example 1. Tables 3 to 9 show the results.

**[Table 3]**

| PDGF-BB cut-off point: 1480 pg/ml (The lower limit at which the difference between 6-month-progression-free survival rates was 20% or more) | | | | | |
|---|---|---|---|---|---|
| Subject | | Number of cases | 6-month-progression -free survival rate (%) | Difference between 6-month-progression -free survival rates (%) | Hazard ratio |
| PDGF-BB hyperexpression group | TSU-68 administration group | 33 | 52 | 21 | 0.64 |
| | TSU-68 non-administration group | 42 | 31 | | |
| PDGF-BB underexpression group | TSU-68 administration group | 16 | 25 | 2 | 0.73 |
| | TSU-68 non-administration group | 8 | 23 | | |

As shown in table 3, when the lower-limit cut-off point (1480 pg/ml) at which the difference between the 6-month-progression-free survival rate for the TSU-68 administration group of the PDGF-BB hyperexpression group and the 6-month-progression-free survival rate for the TSU-68 non-administration group of the PDGF-BB hyperexpression group was 20% or more was selected, substantially equivalent therapeutic effects were confirmed for both the TSU-68 administration group and the TSU-68 non-administration group of the PDGF-BB underexpression group, while on the other hand, therapeutic effects confirmed for the TSU-68 administration group of the PDGF-BB hyperexpression group were obviously more excellent than those confirmed for the TSU-68 non-administration group of the PDGF-BB hyperexpression group.

**[Table 4]**

| PDGF-BB cut-off point: 1740 pg/ml | | | | | |
|---|---|---|---|---|---|
| (The lower limit at which the hazard ratio was 0.6 or less) | | | | | |
| Subject | | Number of cases | 6-m onth-progression -free survival rate (%) | Difference between 6-month-progression -free survival rates (%) | Hazard ratio |
| PDGF-BB hyperexpression group | TSU-68 administration group | 24 | 52 | 25 | 0.59 |
| | TSU-68 non-administration group | 36 | 27 | | |
| PDGF-BB underexpression group | TSU-68 administration group | 25 | 40 | 2 | 0.85 |
| | TSU-68 non-administration group | 14 | 38 | | |

As shown in table 4, when the lower-limit cut-off point (1740 pg/ml) at which the hazard ratio of the TSU-68 administration group of the PDGF-BB hyperexpression group to the TSU-68 non-administration group of the PDGF-BB hyperexpression group was 0.6 or less was selected, substantially equivalent therapeutic effects were confirmed for both the TSU-68 administration group and the TSU-68 non-administration group of the PDGF-BB underexpression group, while on the other hand, therapeutic effects confirmed for the TSU-68 administration group of the PDGF-BB hyperexpression group were obviously more excellent than those confirmed for the TSU-68 non-administration group of the PDGF-BB hyperexpression group.

**[Table 5]**

| PDGF-BB cut-off point: 1960 pg/ml | | | | | |
|---|---|---|---|---|---|
| (The lower limit at which the hazard ratio was 0.6 or less) | | | | | |
| Subject | | Number of cases | 6-month-progression -free survival rate (%) | Difference between 6-month-progression -free survival rates (%) | Hazard ratio |
| PDGF-BB hyperexpression group | TSU-68 administration group | 21 | 52 | 25 | 0.55 |
| | TSU-68 non-administration group | 26 | 27 | | |
| PDGF-BB underexpression group | TSU-68 administration group | 28 | 40 | 7 | 0.84 |
| | TSU-68 non-administration group | 24 | 33 | | |

As shown in table 5, when the upper-limit cut-off point (1960 pg/ml) at which the hazard ratio of the TSU-68 administration group of the PDGF-BB hyperexpression group to the TSU-68 non-administration group of the PDGF-BB hyperexpression group was 0.6 or less was selected, substantially equivalent therapeutic effects were confirmed for both the TSU-68 administration group and the TSU-68 non-administration group of the PDGF-BB underexpression group, while on the other hand, therapeutic effects confirmed for the TSU-68 administration group of the PDGF-BB hyperexpression group were obviously more excellent than those confirmed for the TSU-68 non-administration group of the PDGF-BB hyperexpression group.

**[Table 6]**

| PDGF-BB cut-off point: 2030 pg/ml (The upper limit at which the difference between 6-month-progression-free survival rates was 20% or more) | | | | | |
|---|---|---|---|---|---|
| Subject | | Number of cases | 6-month-progression -free survival rate (%) | Difference between 6-month-progression -free survival rates (%) | Hazard ratio |
| PDGF-BB hyperexpression group | TSU-68 administration group | 20 | 52 | 24 | 0.62 |
| | TSU-68 non-administration group | 24 | 28 | | |
| PDGF-BB underexpression group | TSU-68 administration group | 29 | 42 | 10 | 0 75 |
| | TSU-68 non-administration group | 26 | 32 | | |

As shown in table 6, when the upper-limit cut-off point (2030 pg/ml) at which the difference between the 6-month-progression-free survival rate for the TSU-68 administration group of the PDGF-BB hyperexpression group and the 6-month-progression-free survival rate for the TSU-68 non-administration group of the PDGF-BB hyperexpression group was 20% or more was selected, substantially equivalent therapeutic effects were confirmed for both the TSU-68 administration group and the TSU-68 non-administration group of the PDGF-BB underexpression group, while on the other hand, therapeutic effects confirmed for the TSU-68 administration group of the PDGF-BB hyperexpression group were obviously more excellent than those confirmed for the TSU-68 non-administration group of the PDGF-BB hyperexpression group.

**[Table 7]**

| IL-8 cut-off point: 2.1 pg/ml | | | | | |
|---|---|---|---|---|---|
| (The lower limit at which the hazard ratio was 0.6 or less; the lower limit at which the difference between 6-month-progression-free survival rates was 20% or more) | | | | | |
| Subject | | Number of cases | 6-month-progression -free survival rate (%) | Difference between 6-month-progression -free survival rates (%) | Hazard ratio |
| IL-8 hyperexpression group | TSU-68 administration group | 46 | 48 | 23 | 0.51 |
| | TSU-68 non-administration group | 42 | 25 | | |
| IL-8 underexpression group | TSU-68 administration group | 3 | 0 | -57 | - |
| | TSU-68 non-administration group | 8 | 57 | | |

As shown in table 7, when the lower-limit cut-off point (2.1 pg/ml) at which the difference between the 6-month-progression-free survival rate for the TSU-68 administration group of the IL-8 hyperexpression group and the 6-month-progression-free survival rate for the TSU-68 non-administration group of the IL-8 hyperexpression group was 20% or more and the hazard ratio of the TSU-68 administration group of the IL-8 hyperexpression group to the TSU-68 non-administration group of the IL-8 hyperexpression group was 0.6 or less was selected, substantially equivalent therapeutic effects were confirmed for both the TSU-68 administration group and the TSU-68 non-administration group of the IL-8 underexpression group, while on the other hand, therapeutic effects confirmed for the TSU-68 administration group of the IL-8 hyperexpression group were obviously more excellent than those confirmed for the TSU-68 non-administration group of the IL-8 hyperexpression group.

**[Table 8]**

| IL-8 cut-off point: 6.6 pg/ml | | | | | |
|---|---|---|---|---|---|
| (The upper limit at which the difference between 6-month-progression-free survival rates was 20% or more) | | | | | |
| Subject | | Number of cases | 6-month-progression-free survival rate (%) | Difference between 6-month-progression-free survival rates (%) | Hazard ratio |
| IL-8 hyperexpression group | TSU-68 administration group | 28 | 50 | 20 | 0.51 |
| | TSU-68 non-administratio n group | 23 | 30 | | |
| IL-8 underexpression group | TSU-68 administration group | 21 | 39 | 9 | 0 92 |
| | TSU-68 non-administratio n group | 27 | 30 | | |

As shown in table 8, when the upper-limit cut-off point (6.6 pg/ml) at which the difference between the 6-month-progression-free survival rate for the TSU-68 administration group of the IL-8 hyperexpression group and the 6-month-progression-free survival rate for the TSU-68 non-administration group of the IL-8 hyperexpression group was 20% or more, which is the median of IL-8 expression levels of all cases, was selected, substantially equivalent therapeutic effects were confirmed for both the TSU-68 administration group and the TSU-68 non-administration group of the IL-8 underexpression group, while on the other hand, therapeutic effects confirmed for the TSU-68 administration group of the IL-8 hyperexpression group were obviously more excellent than those confirmed for the TSU-68 non-administration group of the IL-8 hyperexpression group.

**[Table 9]**

| IL-8 cut-off point: 10.5 pg/ml | | | | | |
|---|---|---|---|---|---|
| (The upper limit at which the hazard ratio was 0.6 or less) | | | | | |
| Subject | | Number of cases | 6-month-progression -free survival rate (%) | Difference between 6-month-progression-f ree survival rates (%) | Hazard ratio |
| IL-8 hyperexpression group | TSU-68 administration group | 15 | 56 | 27 | 0.51 |
| | TSU-68 non-administration group | 13 | 29 | | |
| IL-8 underexpression group | TSU-68 administration group | 34 | 41 | 11 | 0.79 |
| | TSU-68 non-administration group | 37 | 30 | | |

As shown in table 9, when the upper-limit cut-off point (10.5 pg/ml) at which the hazard ratio of the TSU-68 administration group of the IL-8 hyperexpression group to the TSU-68 non-administration group of the IL-8 hyperexpression group was 0.6 or less was selected, substantially equivalent therapeutic effects were confirmed for both the TSU-68 administration group and the TSU-68 non-administration group of the IL-8 underexpression group, while on the other hand, therapeutic effects confirmed for the TSU-68 administration group of the IL-8 hyperexpression group were obviously more excellent than those confirmed for the TSU-68 non-administration group of the IL-8 hyperexpression group.

As described above, similar therapeutic effects were confirmed for patients having low PDGF-BB or IL-8 blood levels in the TSU-68 administration group and the TSU-68 non-administration group. Meanwhile, the difference between the 6-month-progression-free survival rate for the TSU-68 administration group and the 6-month-progression-free survival rate for the TSU-68 non-administration group was 22% to 27% and the hazard ratio of the TSU-68 administration group to the TSU-68 non-administration group was 0.51 to 0.68 for patients having high PDGF-BB or IL-8 blood levels. Thus, excellent therapeutic effects were confirmed.

As described above, it has been revealed that remarkably excellent therapeutic effects of TSU-68 can be expected by selecting hepatocellular carcinoma patients who have been treated with TACE using, as an indicator, the PDGF-BB or IL-8 level.

In addition, it has been reported that therapeutic effects of chemotherapy on hepatocellular carcinoma patients who have not received TACE treatment can be predicted using, as an indicator, the level of PDGF-BB, VCAM, or the like (European Journal of Cancer Supplements, 6 (12) :17 #43, 2008, "Assessment of predictive biological markers with an oral angiogenic receptor tyrosine kinase (RTK) inhibitor, TSU-68, in the Phase I/II study for advanced hepatocellular carcinoma (HCC), T. Okusaka et al.). However, when the patient group of the present invention was stratified using the reported VCAM-cut-off point (2370 pg/ml), the difference between the 6-month-progression-free survival rate for the TSU-68 administration group and the 6-month-progression-free survival rate for the TSU-68 non-administration group was not 20% or more, and also the hazard ratio of the TSU-68 administration group to the TSU-68 non-administration group was not 0.6 or less (data not shown). This is probably because a variety of genes of hepatocellular carcinoma patients who have received TACE treatment are expressed more differently than those of hepatocellular carcinoma patients who have not received TACE treatment (Liao X. et al, J Huazhong Univ Sci Technolog Med Sci. 2003; 23 (3): 280-2; Kobayashi N. et al, Liver. 1999 Feb; 19 (1): 25-31). The above results suggest that there are differences between hepatocellular carcinoma patients who have received TACE treatment and hepatocellular carcinoma patients who have not received TACE treatment in terms of groups of molecules that can be used as indicators for predicting therapeutic effects of chemotherapy via TSU-68 administration. Further, the results suggest that not all of molecules involved in angiogenesis can be used as an indicator for predicting therapeutic effects of chemotherapy via TSU-68 administration.

### Industrial Applicability

According to the prediction method of the present invention, chemotherapy with TSU-68 can be adequately provided only to hepatocellular carcinoma patients who have been treated with TAE and who can be expected to obtain therapeutic effects therefrom. Hence, unnecessary chemotherapy can be omitted and thus burdens on patients can be reduced. Further, the present invention is useful in terms of medical cost efficiency.

All publications, patents, and patent applications cited herein are incorporated herein by reference in their entirety.

## Claims

1. A method for predicting therapeutic effects of chemotherapy with an antitumor agent comprising (Z)-5-[(1,2-dihydro-2-oxo-3H-indol-3-ylidene)methyl]2,4-dimethyl-1H-pyrro 1-3-propanoic acid or a salt thereof on a hepatocellular carcinoma patient who has been treated with transarterial embolization, which comprises the following steps (1) to (3):
(1) a step of determining the expression level of PDGF-BB or IL-8 contained in a biological sample collected from the patient;
(2) a step of comparing the expression level of PDGF-BB or IL-8 determined in step (1) with a predetermined corresponding cut-off point; and
(3) a step of predicting that the patient has a high probability of obtaining sufficient therapeutic effects from chemotherapy with an antitumor agent comprising (Z)-5-[(1,2-dihydro-2-oxo-3H-indol-3-ylidene)methyl]2,4-dimethyl-1H-pyrro 1-3-propanoic acid or a salt thereof, if the expression level of PDGF-BB or IL-8 is higher than the cut-off point as a result of comparison in step (2).

2. The method of claim 1, wherein transarterial embolization is transarterial chemoembolization.

3. An antitumor agent comprising (Z)-5-[(1,2-dihydro-2-oxo-3H-indol-3-ylidene)methyl]2,4-dimethyl-1H-pyrro 1-3-propanoic acid or a salt thereof, which is administered to a cancer patient who has been predicted to have a high probability of obtaining sufficient therapeutic effects from chemotherapy with an antitumor agent comprising (Z)-5-[(1,2-dihydro-2-oxo-3H-indol-3-ylidene)methyl]2,4-dimethyl-1H-pyrro 1-3-propanoic acid or a salt thereof according to the method of claim 1 or 2.

4. An antitumor agent kit including the antitumor agent of claim 3 and instructions for use that specify administration of (Z)-5-[(1,2-dihydro-2-oxo-3H-indol-3-ylidene)methyl]2,4-dimethyl-1H-pyrro 1-3-propanoic acid or a salt thereof to a cancer patient who has been predicted to have a high probability of obtaining sufficient therapeutic effects from chemotherapy with an antitumor agent comprising (Z)-5-[(1,2-dihydro-2-oxo-3H-indol-3-ylidene)methyl]2,4-dimethyl-1H-pyrro 1-3-propanoic acid or a salt thereof according to the method of claim 1 or 2.

5. A method for treating hepatocellular carcinoma of a hepatocellular carcinoma patient who has been treated with transarterial embolization, which comprises administering an antitumor agent comprising (Z)-5-[(1,2-dihydro-2-oxo-3H-indol-3-ylidene)methyl]2,4-dimethyl-1H-pyrro 1-3-propanoic acid or a salt thereof to a cancer patient who has been predicted to have a high probability of obtaining sufficient therapeutic effects from chemotherapy with an antitumor agent comprising (Z)-5-[(1,2-dihydro-2-oxo-3H-indol-3-ylidene)methyl]2,4-dimethyl-1H-pyrro 1-3-propanoic acid or a salt thereof according to the method of claim 1 or 2.

6. (Z)-5-[(1,2-dihydro-2-oxo-3H-indol-3-ylidene)methyl]2,4-dimethyl-1H -pyrrol-3-propanoic acid or a salt thereof, which is used for a cancer patient who has been predicted to have a high probability of obtaining sufficient therapeutic effects from chemotherapy with an antitumor agent comprising (Z)-5-[(1,2-dihydro-2-oxo-3H-indol-3-ylidene)methyl]2,4-dimethyl-1H-pyrro 1-3-propanoic acid or a salt thereof according to the method of claim 1 or 2 in order to treat hepatocellular carcinoma of a hepatocellular carcinoma patient who has been treated with transarterial embolization.
